(19) Europäisches Patentamt
European Patent Office
Office européen des brevets

(11) EP 1 622 452 B1

(12) EUROPÄISCHE PATENTSCHRIFT

(45) Veröffentlichungstag und Bekanntmachung des Hinweises auf die Patenterteilung:
**16.03.2011 Patentblatt 2011/11**

(21) Anmeldenummer: **04728332.0**

(22) Anmeldetag: **20.04.2004**

(51) Int Cl.:
***A01N 43/78*** *(2006.01)*

(86) Internationale Anmeldenummer:
**PCT/EP2004/004167**

(87) Internationale Veröffentlichungsnummer:
**WO 2004/095930 (11.11.2004 Gazette 2004/46)**

(54) **WIRKSTOFFKOMBINATIONEN MIT NEMATIZIDEN UND INSEKTIZIDEN EIGENSCHAFTEN BASIEREND AUF TRIFLUORBUTENYL-VERBINDUNGEN**

ACTIVE SUBSTANCE COMBINATION BASED ON TRIFLUOROBUTINYL COMPOUNDS AND EXHIBITING NEMATICIDAL AND INSECTICIDAL PROPERTIES

ASSOCIATIONS DE SUBSTANCES ACTIVES PRESENTANT DES PROPRIETES NEMATICIDES ET INSECTICIDES, A BASE DE COMPOSES TRIFLUOROBUTENYLE

(84) Benannte Vertragsstaaten:
**AT BE BG CH CY CZ DE DK EE ES FI FR GB GR HU IE IT LI LU MC NL PL PT RO SE SI SK TR**

(30) Priorität: **02.05.2003 DE 10319590**

(43) Veröffentlichungstag der Anmeldung:
**08.02.2006 Patentblatt 2006/06**

(73) Patentinhaber: **MAKHTESHIM CHEMICAL WORKS LIMITED**
**84100 Beer Sheva (IL)**

(72) Erfinder:
- **KRAUS, Anton**
**42799 Leichlingen (DE)**
- **ISHIKAWA, Koichi**
**Oyama-shi, Tochigi 323-0820 (JP)**
- **Andersch, Wolfram**
**51469 Bergisch Gladbach (DE)**

(74) Vertreter: **Modiano, Micaela Nadia et al**
**Modiano Josif Pisanty & Staub Ltd**
**Thierschstrasse 11**
**80538 München (DE)**

(56) Entgegenhaltungen:
**WO-A-01/02378 WO-A-03/029231**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Wirkstoffkombinationen, die aus bekannten heterocyclischen Trifluorbutenylen einerseits und bekannten insektiziden Wirkstoffen andererseits bestehen und sehr gut zur Bekämpfung von tierischen Schädlingen wie Insekten, und Nematoden geeignet sind.

Es ist bereits bekannt, dass bestimmte heterocyclische Trifluorbutenyle nematizide Eigenschaften besitzen (WO 01/02378 A1). Eine Wirksamkeit dieser Stoffe gegen Insekten wird nicht berichtet. Es wurde nun gefunden, dass bestimmte heterocyclische Trifluorbutenyle auch eine insektizide Wirkung besitzen. Diese Wirkung ist gut, jedoch nicht in allen Bereichen zufriedenstellend.

Weiterhin ist bekannt, dass zahlreiche Phosphorsäureester, Carbamate, Heterocyclen, OrganozinnVerbindungen, Benzoylharnstoffe und Pyrethroide insektizide und akarizide Eigenschaften besitzen (vgl. z.B. US 2,758,115, US 3,309,266, GB 1,181,657, WO 93/22297 A1, WO 93/10083 A1, DE 26 41 343 A1, EP 347 488 A1, EP 210 487 A1, US 3,264,177 und EP 234 045 A2). Allerdings ist auch die Wirkung dieser Stoffe nicht in allen Belangen befriedigend.

Es wurde nun gefunden, dass die neuen Wirkstoffkombinationen aus zumindest einer Verbindung der Formel (I)

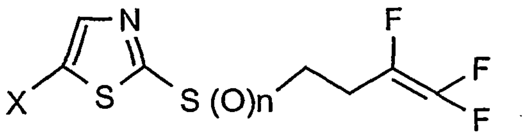

(I)

in welcher

X für Halogen, und

n für 0, 1 oder 2 steht,

("Wirkstoffe der Gruppe 1")
und
zumindest einem Wirkstoff aus der folgenden Gruppe von Wirkstoffen,
Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, *Bacillus popilliae, Bacillus sphaericus, Bacillus subtilis, Bacillus thuringiensis, Bacillus thuringiensis* strain EG-2348, *Bacillus thuringiensis* strain GC-91, *Bacillus thuringiensis* strain NCTC-11821, Baculoviren, *Beauveria bassiania, Beauveria tenella,* Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, *Cydia pomonella* Granuloseviren, Cyfluthrin, Cyhalothrin, Cyhexatin, Cypermethrin, Cyphenothrin (1R-trans-isomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-Cyhalothrin, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid,

Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, *Metharhizium anisopliae, Metharhizium flavoviride,* Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, *Paecilomyces fumosoroseus,* Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, *Verticillium lecanii,* WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo-[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923) sowie der Verbindung der Formel (IIA)

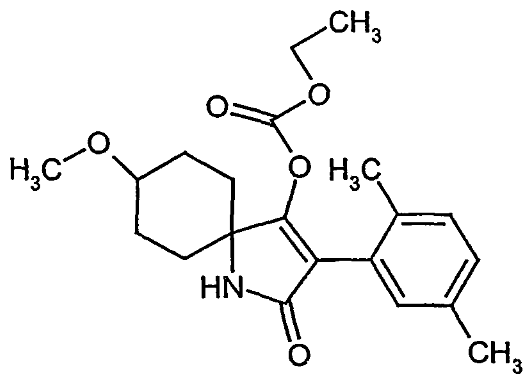

(IIA)

(Carbonsäure, 3-(2,5-dimethylphenyl)-8-methoxy-2-oxo-1-azaspiro[4.5]dec-3-en-4-yl-ethyl-ester, (9C1)) ("Wirkstoffe der Gruppe 2").

sehr gute nematizide, insektizide und akarizide Eigenschaften besitzen.

Überraschenderweise ist die nematizide, insektizide bzw. akarizide Wirkung der erfindungsgemäßen Wirkstoffkombinationen wesentlich höher als die Summe der Wirkungen der einzelnen Wirkstoffe. Es liegt daher ein nicht vorhersehbarer synergistischer Effekt vor und nicht nur eine Wirkungsergänzung.

Die erfindungsgemäßen Wirkstoffkombinationen enthalten neben mindestens einem Wirkstoff der Formel (I) mindestens einen Wirkstoff der Gruppe 2.

Die Wirkstoffe der Gruppe 2 können in die folgenden verschiedene Substanzklassen unterteilt werden, z.B:

**Benzisothiazole,** wie z.B. Benclothiaz; **Benzoylharnstoffe,** wie z.B. Bistrifluron, Chlorfluazuron, Diflubenzuron, DOWCO-439, Fluazuron, Flucycloxuron, Flufenoxuron, Hexaflumuron, Lufenuron, Novaluron, Noviflumuron, Teflubenzuron, Triflumuron; **Biologische Insektizide,** wie z.B. ABG-9008, *Bacillus popilliae, Bacillus sphaericus, Bacillus subtilis, Bacillus thuringiensis, Bacillus thuringiensis* strain EG-2348, *Bacillus thuringiensis* strain NCTC-11821, *Bacillus thuringiensis* strain GC-91, Baculoviren, *Beauveria bassiana, Beauveria tenella, Cydia pomonella* Granuloseviren (CpGV), Entomophthora spp., Granuloseviren, Kernpolyeder-Viren, *Metharhizium anisopliae, Metharhizium flavoviride, Paecilomyces fumosoroseus, Verticillium lecanii,* **Carbamate,** wie z.B. Alanycarb, Aldicarb, Aldoxycarb, Aminocarb, Bendiocarb, Benfuracarb, BPMC, Bufencarb, Butocarboxim, Carbaryl, Carbofuran, Carbosulfan, Cloethocarb, Ethiofencarb, Fenobucarb, Fenoxycarb, Furathiocarb, Isoprocarb, Metam-sodium, Methiocarb, Methomyl, Metolcarb, Metolcarb, Oxamyl, Phosphocarb, Pirimicarb, Promecarb, Propoxur, Thiodicarb, Thiofanox, Trimethacarb, XMC, Xylylcarb; **Dinitrophenole,** wie z.B. Binapacryl, Dinobuton, Dinocap; **Diphenylether,** wie z.B. Difenolan, Dofenapyn, Pyriproxyfen; **Ester,** wie z.B. Cycloprene, Gossyplure, Hydroprene, Kinoprene, Methoprene, Docusat-sodium, Spirodiclofen, Spiromesifen; **Indenooxadiazincarboxamide,** wie z.B. Indoxacarb; **Makrolide,** wie z.B. Abamectin, Avermectin, Emamectin, Emamectin-Benzoate, Ivermectin, Milbemectin, Milbemycin, Moxidectin, Spinosad, Thuringiensin; **Neo-Nicotinoide,** wie z.B. Acetamiprid, AKD 1022, Clothianidin, Dinetofuran, Imidacloprid, Nitenpyram, Thiacloprid, Thiamethoxam; **Phosphate,** wie z.B. Bromfenvinfos (-methyl), Chlorfenvinphos, Dichlorvos, Dicrotophos, Dimethylvinphos, Heptenophos, Mevinphos, Monocrotophos, Naled, Phosphamidon, Propaphos, Temivinphos, Tetrachlorvinphos; **Phosphoramidate,** wie z.B. Fenamiphos, Isofenphos; **Phosphoramidothioate,** wie z.B. Acephate, Methamidophos, Propetamphos; **Phthalamide,** wie z.B. N2-[1,1-Dimethyl-2(methylsulfonyl)-ethyl]-3-iodo-N1-[2-methyl-4-[1,2,2,2-tetrafluoro-1-(trifluoromethyl)ethyl]phenyl-1,2-benzenedicarboxamide (CAS-Reg.-Nr. 272451-65-7, vgl. EP 0 919 542 A2), Pyrazole, wie z.B. Acetoprole, Ethiprole, Fenpyroximate, Fipronil, Vaniliprole; **Pyrazolearbozamide,** wie z.B. Fenyprad, Tebufenpyrad, Tolfenpyrad; **Pyrethroide und Pyrethroid-Analoge,** wie z.B. Resmethrin, Acrinathrin, Allethrin (IR-Isomer), Alpha-Cypermethrin, Beta-, Cyfluthrin, Beta-Cypermethrin, Bifenthrin, Bioallethrin, Bioallethrin (S-cyclopentyl-Isomer), Bioethanomethrin, Biopermethrin, Bioresmethrin, Brofenprox, Chloethocarb, Chlovaporthrin, Cis-Cypermethrin, Cis-Resmethrin, Clocythrin, Cycloprothrin, Cyfluthrin, Cyhalothrin, Cypermethrin, Cyphenothrin (1R-trans-, somer), Deltamethrin, Dimefluthrin, Eflusilanate, Empenthrin (1R-Isomer), Esfenvalerate, Etofenprox, Fenfluthrin, Fenpropathrin, Fenpyrithrin, Fenvalerate, Flubrocythrinate, Flubrocythrinate, Flucythrinate, Flufenprox, Flufenprox, Flumethrin, Fluvalinate, Fubfenprox, Gamma-Cyhalothrin, Halfenprox, Imiprothrin, Kadethrin, Lambda-Cyhalothrin, Metofluthrin, MIT-800, Permethrin, Phenothrin (1R-trans-Isomer), Prallethrin, Profluthrin, Protrifenbute, Pyresmethrin, Pyrethrum, RU-12457, RU-15525, Silafluofen, Tau-Fluvalinate, Tefluthrin, Tetramethrin (1R-Isomer), Theta-Cypermethrin, Tralocythrin, Tralomethrin, Transfluthrin, Zeta-Cypermethrin; **Pyridazinone,** wie z.B. Butylpyridaben, NC-170, NC-184, NC-194, NC-196, Pyridaben, Pyridaphenthion, Pyridathion; **Pyrrole,** wie z.B. Chlorfenapyr; **Quinazoline,** wie z.B. Fenazaquin; **Thiophosphate** und **Dithiophosphate,** wie z.B. Azamethiphos, Azinphos-ethyl, Azinphos-methyl, Bromophos-ethyl, Butathiofos, Cadusafos, Carbophenothion, Chlorethoxyfos, Chlormephos, Chlorpyrifos, Chlorpyrifos, Chlorpyrifos-ethyl, Chlorpyrifos-methyl, Coumaphos, Cyanophos, Demeton, Demeton-S-methyl, Demeton-S-methyl-sulphon, Dialifos, Diazinon, Dichlofenthion, Dimethoate, Disulfoton, Ethion, Ethoprophos, Etrimfos, Fenitrothion, Fensulfothion, Fenthion, Flupyrazofos, Fonofos, Formothion, Fosmethilan, Iodofenphos, Iprobenfos, Isazofos, Isoxathion, Malathion, Mecarbam, Mesulfenfos, Methacrifos, Methidathion, Omethoate, Oxydemeton-methyl, Parathion-methyl, Phenthoate, Phorate, Phosalone, Phosmet, Phosphocarb, Phoxim, Pirimiphos-ethyl, Pirimiphos-, methyl, Profenofos, Prothiofos, Prothoate, Pyraclofos, Pyridaphenthion, Pyridathion, Quinalphos, Sulfotep, Sulprofos, Tebuprimifos, Temephos, Terbufos, Thiatriphos, Thiometon, Triazophos, Vamidothion; **Thiophosphonate,** wie z.B. Cyanofenphos, EPN, Fosthiazate; **Thiosulfonate,** wie z.B. Bensultap, Thiosultap-sodium; **Thioharnstoffe,** wie z.B. Diafenthiuron; Triazine, wie z.B. Cyromazine, Pymetrozine; **Triazolcarboxamide,** wie z.B. Triazamate, Triazuron.

Im Rahmen der vorliegenden Erfindung wurde festgestellt, dass erfindungsgemäße Kombinationen aus einem Wirkstoff der Gruppe 1 und einem oder mehreren Wirkstoffen aus einer der vorstehend genannten Substanzklassen eine synergistische Wirkungssteigerung im Vergleich zu den jeweils alleine eingesetzten Wirkstoffen zeigen.

So sind insbesondere Wirkstoffkombinationen enthaltend zumindest einen Wirkstoff aus der Gruppe 1 und zumindest einen Wirkstoff aus einer oder mehrerer der vorstehend definierten Stoffklassen der Carbamate, Neo-Nicotinoide, Pyrazole, Makrolide, Thiophosphate bzw. Dithiophosphate, oder der Pyrethroide bzw. Pyrethroid-Analoga Gegenstand der vorliegenden Erfindung.

Bestimmte Stoffklassen zeichnen sich auch durch einen gemeinsamen Wirkungsmechanismus bzw. einen gemeinsamen Wirkort aus:

"Carbamate" sowie "Thiophosphate" entfalten ihre neurotoxische Wirkung durch Hemmung des Enzyms Acetylcholinesterase, das bei der Nervenreizleitung eine eminent wichtige Rolle spielt: es zerstört mit hoher Reaktionsgeschwindigkeit den Botenstoff Acetylcholin, der die Erregung von einem Neuron an ein anderes weitergibt. Hemmt man das Enzym, so häuft sich Acetylcholin an und das gesamte Neuronensystem gelangt in einen Zustand der Übererregung.

Die "Neo-Nicotinoide" können an Rezeptormoleküle im Nervensystem andocken, die normalerweise Acetylcholin, den Botenstoff, der die Erregung von einem Neuron an ein anderes weitergibt, akzeptieren. Die Wirkstoffe blockieren die Acetylcholin-Rezeptoren irreversibel und stören damit entscheidend die physiologischen Prozesse im Insekt.

"Pyrethroide" bzw. "Pyrethroid-Analoga" verzögern das Schließen des Natriumkanals an der Plasmamembran

der Nervenzelle. Damit wird der physiologische Ablauf der Repolarisation und der Aufbau eines ausreichend negativen Ruhemembranpotentials behindert. Sie verursachen außerdem eine Erhöhung der Calciumkonzentrationen im präsynaptischen Neuron durch Hemmung der calcium- und magnesiumabhängigen ATPase und des calciumbindenden Proteins Calmodulin. Dies bewirkt eine erhöhte Freisetzung von Neurotransmittern und eine verstärkte Depolarisation der postsynaptischen Membran. Schließlich verhindern die Pyrethroide den GABA-induzierten Chlorideinstrom. Letzteres wird auch bei Insektiziden des Cyclodien Typs beobachtet.

"Pyrazole", die gelegentlich auch "Fiprole" genannt werden, wirken auf den GABA (gammaamino-butric acid) Rezeptor der Insekten, indem sie den Durchtritt von Chloridionen verhindern und auf diese Weise den Zusammenbruch des zentralen Nervensystems herbeiführen. Diese Wirkungsweise entspricht auch der von Cyclodienen.

"Benzoylharnstoffe" wirken während des Larvenstadiums der meisten Insekten indem sie die Biosynthese des Chitins stören. Typische Effekte z.B. sind die Zerstörung oder Missbildung der Cuticula.

Bevorzugt sind weiterhin Wirkstoffkombinationen wie vorstehend beschrieben, die zumindest eine Verbindung der Formel (I) enthalten, in welcher

X für Fluor, Chlor oder Brom steht, und
n für 0 oder 2 steht.

Besonders bevorzugt sind weiterhin Wirkstoffkombinationen wie vorstehend beschrieben, die zumindest eine Verbindung der Formel (I) enthalten, in welcher

X für Fluor oder Chlor steht, und
n für 2 steht.

Insbesondere bevorzugt sind Wirkstoffkombinationen wie vorstehend beschrieben, enthaltend eine Verbindung der Formel (IA)

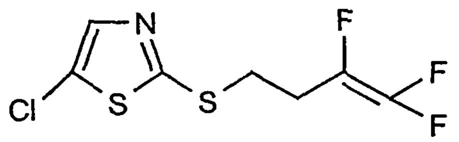

(IA)

und einen Wirkstoff der Gruppe 2,
bevorzugt einen Wirkstoff aus der Reihe Aldicarb, Alanycarb, Aldoxycarb, Aminocarb, Bendiocarb, Benfuracarb, BPMC, Bufencarb, Butocarboxim, Carbaryl, Carbofuran, Carbosulfan, Cloethocarb, Ethiofencarb, Fenobucarb, Fenoxycarb, Furathiocarb, Isoprocarb, Metam-sodium, Methiocarb, Methomyl, Metolcarb, Metolcarb, Oxamyl, Phosphocarb, Pirimicarb, Promecarb, Propoxur, Thiodicarb, Thiofanox, Trimethacarb, XMC, Xylylcarb ("Carbamate"), oder
einen Wirkstoff aus der Reihe Clothianidin, Acetamiprid, AKD 1022, Dinetofuran, Imidacloprid, Nitenpyram, Thiacloprid, Thiamethoxam ("Neo-Nicotinoide"), oder
einen Wirkstoff aus der Reihe Fipronil, Acetoprole, Ethiprole, Fenpyroximate, Vaniliprole ("Pyrazole"), oder
einen Wirkstoff aus der Reihe Spinosad, Abamectin, Avermectin, Emamectin, Emamectin-Benzoate, Ivermectin, Milbemectin, Milbemycin, Moxidectin, Thuringiensin ("Makrolide"), oder
einen Wirkstoff aus der Reihe Tebupirimfos, Azamethiophos, Azinphos-ethyl, Azinphos-methyl, Bromophos-ethyl, Butathiofos, Cadusafos, Carbophenothion, Chlorethoxyfos, Chlorpyrifos, Chlorpyrifos, Chlorpyrifos-ethyl, Chlorpyrifos-methyl, Coumaphos, Cyanophos, Demeton, Demeton-S-methyl, Demeton-S-methyl-sulphon, Dialifos, Diazinon, Dichlofenthion, Dimethoate, Disulfoton, Ethion, Ethoprophos, Etrimfos, Fenitrothion, Fensulfothion, Fenthion, Flupyrazofos, Fonofos, Formothion, Fosmethilan, Iodofenphos, Iprobenfos, Isazofos, Isoxathion, Malathion, Mecarbam, Mesulfenfos, Methacrifos, Methidathion, Omethoate, Oxydemeton-methyl, Parathion-methyl, Phenthoate, Phorate, Phosalone, Phosmet, Phosphocarb, Phoxim, Pirimiphos-ethyl, Pirimiphos-, methyl, Profenofos, Prothiofos, Prothoate, Pyraclofos, Pyridaphenthion, Pyridathion, Quinalphos, Sulfotep, Sulprofos, Temephos, Terbufos, Thiatriphos, Thiometon, Triazophos, Vamidothion ("Thiophosphate" bzw. "Dithiophosphate"), oder
einen Wirkstoff aus der Reihe Tefluthrin, Resmethrin, Acrinathrin, Allethrin (IR-Isomer), Alpha-Cypermethrin, Beta-, Cyfluthrin, Beta-Cypermethrin, Bifenthrin, Bioallethrin, Bioallethrin (S-cyclopentyl-Isomer), Bioethanomethrin, Biopermethrin, Bioresmethrin, Brofenprox, Chloethocarb, Chlovaporthrin, Cis-Cypermethrin, Cis-Resmethrin, Clocythrin, Cycloprothrin, Cyfluthrin, Cyhalothrin, Cypermethrin, Cyphenothrin (1R-trans-, somer), Deltamethrin, Dimefluthion, Eflusilanate, Empenthrin (1R-Isomer), Esfenvalerate, Etofenprox, Fenfluthrin, Fenpropathrin, Fenpyrithrin, Fenvalerate, Flubrocythrinate, Flubrocythrinate, Flucythrinate, Flufenprox, Flufenprox, Flumethrin, Fluvalinate, Fubfenprox, Gamma-Cyhalothrin, Halfenprox, Imiprothrin, Kadethrin, Lambda-Cyhalothrin, Metofluthrin, MIT-800, Permethrin, Phenothrin (1R-trans-Isomer), Prallethrin, Profluthrin, Protrifenbute, Pyresmethrin, Pyrethrum, RU-12457, RU-15525, Silafluofen, Tau-Fluvalinate, Tetramethrin (1R-Isomer), Theta-Cypermethrin, Tralocythrin, Tralomethrin, Transfluthrin, Zeta-Cypermethrin ("Pyrethroide" und "Pyrethroid-Analoga").

Insbesondere bevorzugt sind Wirkstoffkombinationen wie vorstehend beschrieben, enthaltend eine Verbindung der Formel (IB)

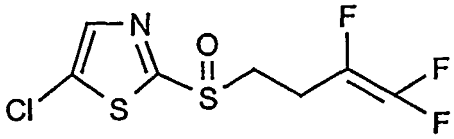 (IB)

und einen Wirkstoff der Gruppe 2,
bevorzugt einen Wirkstoff aus der Reihe Alanycarb, Aldicarb, Aldoxycarb, Aminocarb, Bendiocarb, Benfuracarb, BPMC, Bufencarb, Butocarboxim, Carbaryl, Carbofuran, Carbosulfan, Cloethocarb, Ethiofencarb, Fenobucarb, Fenoxycarb, Furathiocarb, Isoprocarb, Metam-sodium, Methiocarb, Methomyl, Metolcarb, Metolcarb, Oxamyl, Phosphocarb, Pirimicarb, Promecarb, Propoxur, Thiodicarb, Thiofanox, Trimethacarb, XMC, Xylylcarb ("Carbamate"), oder
einen Wirkstoff aus der Reihe Acetamiprid, AKD 1022, Clothianidin, Dinetofuran, Imidacloprid, Nitenpyram, Thiacloprid, Thiamethoxam ("Neo-Nicotinoide"), oder
einen Wirkstoff aus der Reihe Acetoprole, Ethiprole, Fenpyroximate, Fipronil, Vaniliprole ("Pyrazole"), oder
einen Wirkstoff aus der Reihe Abamectin, Avermectin, Emamectin, Emamectin-Benzoate, Ivermectin, Milbemectin, Milbemycin, Moxidectin, Spinosad, Thuringiensin ("Makrolide"), oder
einen Wirkstoff aus der Reihe Azamethiophos, Azinphos-ethyl, Azinphos-methyl, Bromophos-ethyl, Butathiofos, Cadusafos, Carbophenothion, Chlorethoxyfos, Chlorpyrifos, Chlorpyrifos, Chlorpyrifos-ethyl, Chlorpyrifos-methyl, Coumaphos, Cyanophos, Demeton, Demeton-S-methyl, Demeton-S-methyl-sulphon, Dialifos, Diazinon, Dichlofenthion, Dimethoate, Disulfoton, Ethion, Ethoprophos, Etrimfos, Fenitrothion, Fensulfothion, Fenthion, Flupyrazofos, Fonofos, Formothion, Fosmethilan, Iodofenphos, Iprobenfos, Isazofos, Isoxathion, Malathion, Mecarbam, Mesulfenfos, Methacrifos, Methidathion, Omethoate, Oxydemeton-methyl, Parathion-methyl, Phenthoate, Phorate, Phosalone, Phosmet, Phosphocarb, Phoxim, Pirimiphos-ethyl, Pirimiphos-, methyl, Profenofos, Prothiofos, Prothoate, Pyraclofos, Pyridaphenthion, Pyridathion, Quinalphos, Sulfotep, Sulprofos, Tebupirimfos, Temephos, Terbufos, Thiatriphos, Thiometon, Triazophos, Vamidothion ("Thiophosphate"), oder
einen Wirkstoff aus der Reihe Resmethrin, Acrinathrin, Allethrin (1R-Isomer), Alpha-Cypermethrin, Beta-, Cyfluthrin, Beta-Cypermethrin, Bifenthrin, Bioallethrin, Bioallethrin (S-cyclopentyl-Isomer), Bioethanomethrin, Biopermethrin, Bioresmethrin, Brofenprox, Chloethocarb, Chlovaporthrin, Cis-Cypermethrin, Cis-Resmethrin, Clocythrin, Cycloprothrin, Cyfluthrin, Cyhalothrin, Cypermethrin, Cyphenothrin (1R-trans-, somer), Deltamethrin, Dimefluthion, Eflusilanate, Empenthrin (1R-Isomer), Esfenvalerate, Etofenprox, Fenfluthrin, Fenpropathrin, Fenpyrithrin, Fenvalerate, Flubrocythrinate, Flubrocythrinate, Flucythrinate, Flufenprox, Gamma-Cyhalothrin, Flufenprox, Flumethrin, Fluvalinate, Fubfenprox, Halfenprox, Imiprothrin, Kadethrin, Lambda-Cyhalothrin, Metofluthrin, MIT-800, Permethrin, Phenothrin (1R-trans-Isomer), Prallethrin, Profluthrin, Protrifenbute, Pyresmethrin, Pyrethrum, RU-12457, RU-15525, Silafluofen, Tau-Fluvalinate, Tefluthrin, Tetramethrin (1R-Isomer), Theta-Gypermethrin, Tralocythrin, Tralomethrin, Transfluthrin, Zeta-Cypermethrin ("Pyrethroide" und "Pyrethroid-Analoga").

Insbesondere bevorzugt sind Wirkstoffkombinationen wie vorstehend beschrieben, enthaltend eine Verbindung der Formel (IC)

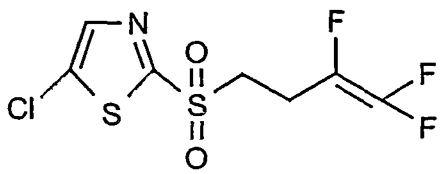 (IC)

und einen Wirkstoff der Gruppe 2,
bevorzugt einen Wirkstoff aus der Reihe Alanycarb, Aldicarb, Aldoxycarb, Aminocarb, Bendiocarb, Benfuracarb, BPMC, Bufencarb, Butocarboxim, Carbaryl, Carbofuran, Carbosulfan, Cloethocarb, Ethiofencarb, Fenobucarb, Fenoxycarb, Furathiocarb, Isoprocarb, Metam-sodium, Methiocarb, Methomyl, Metolcarb, Metolcarb, Oxamyl, Phosphocarb, Pirimicarb, Promecarb, Propoxur, Thiodicarb, Thiofanox, Trimethacarb, XMC, Xylylcarb ("Carbamate"), oder
einen Wirkstoff aus der Reihe Acetamiprid, AKD 1022, Clothianidin, Dinetofuran, Imidacloprid, Nitenpyram, Thiacloprid, Thiamethoxam ("Neo-Nicotinoide"), oder
einen Wirkstoff aus der Reihe Acetoprole, Ethiprole, Fenpyroximate, Fipronil, Vaniliprole ("Pyrazole"), oder
einen Wirkstoff aus der Reihe Abamectin, Avermectin, Emamectin, Emamectin-Benzoate, Ivermectin, Milbemectin, Milbemycin, Moxidectin, Spinosad, Thuringiensin ("Makrolide"), oder
einen Wirkstoff aus der Reihe Azamethiophos, Azinphos-ethyl, Azinphos-methyl, Bromophos-ethyl, Butathiofos, Cadusafos, Carbophenothion, Chlorethoxyfos, Chlorpyrifos, Chlorpyrifos, Chlorpyrifos-ethyl, Chlorpyrifos-methyl, Coumaphos, Cyanophos, Demeton, Demeton-S-methyl, Demeton-S-methyl-sulphon, Dialifos, Diazinon, Dichlofenthion, Dimethoate, Disulfoton, Ethion, Ethoprophos, Etrimfos, Fenitrothion, Fensulfothion, Fenthion, Flupyrazofos, Fonofos, Formothion, Fosmethilan, Iodofenphos, Iprobenfos, Isazofos, Isoxathion, Malathion, Mecarbam, Mesulfenfos, Methacrifos, Methidathion, Omethoate, Oxydemeton-methyl, Parathion-methyl, Phenthoate, Phorate, Phosalone, Phosmet, Phosphocarb, Phoxim, Pirimiphos-ethyl, Pirimiphos-, methyl, Profenofos, Prothiofos, Prothoate, Pyraclofos, Pyridaphenthion, Pyridathion, Quinalphos, Sulfotep, Sulprofos, Tebupirimfos, Temephos, Terbufos, Thiatriphos, Thiometon, Triazophos, Vamidothion ("Thiophosphate" bzw. "Diothiophosphate"), oder
einen Wirkstoff aus der Reihe Resmethrin, Acrinathrin, Allethrin (1R-Isomer), Alpha-Cypermethrin, Beta-, Cyfluthrin, Beta-Cypermethrin, Bifenthrin, Bioallethrin, Bioallethrin (S-cyclopentyl-Isomer), Bioethanomethrin, Biopermethrin, Bioresmethrin, Brofenprox, Chloethocarb, Chlovaporthrin, Cis-Cypermethrin, Cis-Resmethrin, Clocythrin, Cycloprothrin, Cyfluthrin, Cyhalothrin, Cypermethrin, Cyphenothrin (1R-trans-, somer), Deltamethrin, Dimefluthion, Eflusilanate, Empenthrin (1R-Isomer), Esfenvalerate, Etofenprox, Fenfluthrin, Fenpropathrin, Fenpyrithrin, Fenvalerate, Flubrocythrinate, Flubrocythrinate, Flucythrinate, Flufenprox, Flufenprox, Flumethrin, Fluvalinate, Fubfenprox, Gamma-Cyhalothrin, Halfenprox, Imiprothrin, Kadethrin, Lambda-Cyhalothrin, Metofluthrin, MIT-800, Permethrin, Phenothrin (1R-trans-Isomer), Prallethrin, Profluthrin, Protrifenbute, Pyresmethrin, Pyrethrum, RU-12457, RU-15525, Silafluofen, Tau-Fluvalinate, Tefluthrin, Tetramethrin (1R-Isomer), Theta-Cypermethrin, Tralocythrin, Tralomethrin, Transfluthrin, Zeta-Cypermethrin ("Pyrethroide" und "Pyrethroid-Analoga").

Besonders bevorzugte erfindungsgemäße Kombinationen sind in der nachfolgenden Tabelle gezeigt.

**Tabelle 1**

| Wirkstoff der Gruppe 1 | Wirkstoff der Gruppe 2 |
|---|---|
| (IA) | Aldicarb |
| (IB) | Aldicarb |
| (IC) | Aldicarb |
| (IA) | Clothianidin |
| (IB) | Clothianidin |
| (IC) | Clothianidin |
| (IA) | Fipronil |
| (IB) | Fipronil |
| (IC) | Fipronil |
| (IA) | Imidacloprid |
| (IB) | Imidacloprid |
| (IC) | Imidacloprid |
| (IA) | Spinosad |
| (IB) | Spinosad |
| (IC) | Spinosad |
| (IA) | Tebupirimfos |
| (IB) | Tebupirimfos |
| (IC) | Tebupirimfos |
| (IA) | Tefluthrin |

(fortgesetzt)

| Wirkstoff der Gruppe 1 | Wirkstoff der Gruppe 2 |
|---|---|
| (IB) | Tefluthrin |
| (IC) | Tefluthrin |
| (IA) | (IIA) |
| (IB) | (IIA) |
| (IC) | (IIA) |
| (IA) | Chlorethoxyfos |
| (IB) | Chlorethoxyfos |
| (IC) | Chlorethoxyfos |
| (IA) | Ethiprole |
| (IB) | Ethiprole |
| (IC) | Ethiprole |
| (IA) | Thiamethoxam |
| (IB) | Thiamethoxam |
| (IC) | Thiamethoxam |
| (IA) | Carbofuran |
| (IB) | Carbofuran |
| (IC) | Carbofuran |
| (IA) | Terbufos |
| (IB) | Terbufos |
| (IC) | Terbufos |
| (IA) | Carbosulfan |
| (IB) | Carbosulfan |
| (IC) | Carbosulfan |
| (IA) | Furathiocarb |
| (IB) | Furathiocarb |
| (IC) | Furathiocarb |
| (IA) | Cadusafos |
| (IB) | Cadusafos |
| (IC) | Cadusafos |

Die Wirkstoffkombinationen können darüber hinaus auch weitere fungizid, akarizid oder insektizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im allgemeinen enthalten die erfindungsgemäßen Kombinationen Wirkstoffe der Formel (I) und den Mischpartner in den in der nachfolgenden Tabelle angegeben bevorzugten Mischungsverhältnissen, wobei die Mischungsverhältnisse basieren auf Gewichtsverhältnissen. Das Verhältnis ist zu verstehen als Wirkstoff der Formel (I):Mischpartner

**Tabelle 2**

| bevorzugtes Mischungsverhältnis |
|---|
| 2:1 bis 1:1000 |
| 10:1 bis 1:10 |
| 20:1 bis 1:5 |
| 50:1 bis 1:5 |
| 100:1 bis 1:5 |
| 1000 : 1 bis 1: 2 |

Die erfindungsgemäßen Wirkstoffkombinationen eignen sich zur Bekämpfung von tierischen Schädlingen, vorzugsweise Arthropoden und Nematoden, insbesondere Nematoden und Insekten, die in der Landwirtschaft, der Tiergesundheit, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie

natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Auch eine Mischung mit anderen bekannten Wirkstoffen, wie Herbiziden oder mit Düngemitteln und Wachstumsregulatoren ist möglich.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die erfmdungsgemäßen Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffkombinationen eine wirtschaftlichere und einfachere Tierhaltung möglich ist.

Die Anwendung der erfindungsgemäßen Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie Sirex juvencus, Urocerus gigas, Urocerus gigas taignus, Urocerus augur.

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des

Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällern vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Zugleich können die erfindungsgemäßen Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder

Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Wirkstoffkombinationen eine hervorragende Antifouling (Antibewuchs)-Wirkung aufweisen.

Durch Einsatz der erfindungsgemäßen Wirkstoffkombinationen kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie

2-tent.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie z.B. Azaconazole, Cyproconazole, Epoxyconazole, Hexaconazole, Metconazole, Propiconazole und Tebuconazole;

Molluskizide wie

Fentinacetat, Metaldehyd, Methiocarb, Niclosamid, Thiodicarb und Trimethacarb;

oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-S-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoffkombinationen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch

in Self-Polishing-Antifouling-Systemen können die erfindungsgemäßen Verbindungen oder die oben genannten Mischungen eingearbeitet werden.

Die Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft, Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhiozome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus *Bacillus thuringiensis* (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut®

(z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Zink® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfmdungsgemäß mit den erfindungsgemäßen Wirkstoffmischungen behandelt werden. Die bei den Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die gute insektizide, akarizide und nematizide Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt immer dann vor, wenn die Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann nach S.R. Colby, Weeds 15 (1967), 20-22) wie folgt berechnet werden:

Wenn

X den Abtötungsgrad, ausgedrückt in % der unbehandelten Kontrolle, beim Einsatz des Wirkstoffes A in einer Aufwandmenge von <u>m</u> g/ha oder in einer Konzentration von <u>m</u> ppm bedeutet,

Y den Abtötungsgrad, ausgedrückt in % der unbehandelten Kontrolle, beim Einsatz des Wirkstoffes B in einer Aufwandmenge von <u>n</u> g/ha oder in einer Konzentration von n ppm bedeutet und

E den Abtötungsgrad, ausgedrückt in % der unbehandelten Kontrolle, beim Einsatz der Wirkstoffe A und B in Aufwandmengen von <u>m</u> und <u>n</u> g/ha oder in einer Konzentration von <u>m</u> und <u>n</u> ppm bedeutet,

dann ist

$$E = X + Y - \frac{X \cdot Y}{100}$$

Ist der tatsächliche insektizide Abtötungsgrad größer als berechnet, so ist die Kombination in ihrer Abtötung überadditiv, d.h. es liegt ein synergistischer Effekt vor. In diesem Fall muss der tatsächlich beobachtete Abtötungsgrad größer sein als der aus der oben angeführten Formel errechnete Wert für den erwarteten Abtötungsgrad (E).

<u>**Beispiele**</u>

<u>**Beispiel A**</u>

**Phaedon-Larven-Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Larven des Meerrettichblattkäfers (*Phaedon cochleariae*) besetzt, solange die Blätter noch feucht sind. Nach der gewünschten Zeit wird die Abtötung in % bestimmt. Dabei bedeutet 100 %, dass alle Käferlarven

abgetötet wurden; 0 % bedeutet, dass keine Käferlarven abgetötet wurden. Die ermittelten Abtötungswerte verrechnet man nach der Colby-Formel (siehe Blatt 34).

Bei diesem Test zeigte die folgende Wirkstoffkombination gemäß vorliegender Anmeldung eine synergistisch verstärkte Wirksamkeit im Vergleich zu den einzelnen angewendeten Wirkstoffen (gef.* = im Test gefundene Wirkung; ber.** = nach Colby berechnete Wirkung):

**Tabelle 3: (IC) + Tefluthrin**

| Wirkstoff | Wirkstoffkonzentration [ppm] | Abtötungsgrad [%] nach 3 Tagen |
|---|---|---|
| (IC) | 500 | 0 |
| Tefluthrin | 4 | 75 |
| (IC) + Tefluthrin (125:1) | 500+4 | gef.*:100<br>ber.**: 75 |

**Tabelle 4: (IC) + Aldicarb**

| Wirkstoff | Wirkstoffkonzentration [ppm] | Abtötungsgrad [%] nach 3 Tagen |
|---|---|---|
| (IC) | 500 | 0 |
| Aldicarb | 20 | 35 |
| (IC) + Aldicarb (25:1) | 500 + 20 | gef.*: 75<br>ber.**: 35 |

**Tabelle 5: (IC) + Clothianidin**

| Wirstoff | Wirkstoffkonzentration [ppm] | Abtötungsgrad [%] nach 3 Tagen |
|---|---|---|
| (IC) | 500 | 0 |
| Clothianidin | 4 | 15 |
| (IC) + Clothianidin (125:1) | 500 + 4 | gef.*: 75<br>ber.**: 15 |

**Tabelle 6: (IC) + Imidacloprid**

| Wirkstoff | Wirkstoffkonzentration [ppm] | Abtötungsgrad [%] nach 3 Tagen |
|---|---|---|
| (IC) | 500 | 0 |
| Imidacloprid | 20 | 45 |
| (IC) + Imidacloprid (25:1) | 500 + 20 | gef.*: 80<br>ber.**: 45 |

**Beispiel B**

**Plutella-Test, sensibler Stamm**

Lösungsmittel: 7 Gewichtsteile Dimethylformamid

Emulgator: 2 Gewichtsteile Alkylarylpolyglykolether

Zur Herstellung einer zweckmäßigen Wirkstoffzubereitung vermischt man 1 Gewichtsteil Wirkstoff mit den

angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen der Kohlschabe (*Plutella xylostella,* sensibler Stamm) besetzt, solange die Blätter noch feucht sind. Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 34).

Bei diesem Test zeigte die folgende Wirkstoffkombination gemäß vorliegender Anmeldung eine synergistisch verstärkte Wirksamkeit im Vergleich zu den einzelnen angewendeten Wirkstoffen (gef.* = im Test gefundene Wirkung; ber.** = nach Colby berechnete Wirkung):

**Tabelle 7: (IC) + Tefluthrin**

| Wirkstoff | Wirkstoffkonzentration [ppm] | Abtötungsgrad [%] nach 3 Tagen |
|---|---|---|
| **(IC)** | 500 | 0 |
| **Teflutbrin** | 0,16 | 20 |
| **(IC) + Tefluthrin (3125:1)** | 500 + 0,16 | gef.*: 65<br>ber.**: 20 |

**Tabelle 8: (IC) + Aldicarb**

| Wirkzstoff | Wirkstoffkonzentration [ppm] | Abtötungsgrad [%] nach 6 Tagen |
|---|---|---|
| **(IC)** | 500 | 20 |
| **Aldicarb** | 20 | 0 |
| **(IC) + Aldicarb (25:1)** | 500 + 20 | gef.*: 50<br>ber.**: 20 |

**Tabelle 9: (IC) + Imidacloprid**

| Wirkstoff | Wirkstoffkonzentration [ppm] | Abtötungsgrad [%] nach 6 Tagen |
|---|---|---|
| **(IC)** | 500 | 0 |
| **Imidacloprid** | 20 | 5 |
| **(IC) + Imidacloprid (25:1)** | 500 + 20 | gef.*: 65<br>ber.**: 5 |

**Tabelle 10: (IC) + Tebupirimfos**

| Wirkstoff | Wirkstoffkonzentration [ppm] | Abtötungsgrad [%] nach 3 Tagen |
|---|---|---|
| **(IC)** | 500 | 5 |
| **Tebupirimfos** | 0,8 | 0 |
| **(IC) + Tebupirimfos (625:1)** | 500 + 0,8 | gef.*: 40<br>ber.**: 5 |

**Tabelle 11: (IC) + (IIA)**

| Wirkstoff | Wirkstoffkonzentration [ppm] | Abtötungsgrad [%] nach 3 Tagen |
|---|---|---|
| **(IC)** | 500 | 5 |
| **(IIA)** | 4 | 95 |

(fortgesetzt)

| Wirkstoff | Wirkstoffkonzentration [ppm] | Abtötungsgrad [%] nach 3 Tagen |
|---|---|---|
| **(IC) + (IIA) (125:1)** | 500+4 | gef.*: 100<br>ber.**: 95,25 |

**Beispiel C**

**Spodoptera frugiperda-Test**

Lösungsmittel: 7 Gewichtsteile Dimethylformamid

Emulgator: 2 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen des Heerwurms (*Spodoptera frugiperda*) besetzt, solange die Blätter noch feucht sind. Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 34).

Bei diesem Test zeigte die folgende Wirkstoffkombination gemäß vorliegender Anmeldung eine synergistisch verstärkte Wirksamkeit im Vergleich zu den einzelnen angewendeten Wirkstoffen (gef.* = im Test gefundene Wirkung; ber.** = nach Colby berechnete Wirkung):

**Tabelle 12: (IC) + Tefluthrin**

| Wirkstoff | Wirkstoffkonzentration [ppm] | Abtötungsgrad [%] nach 3 Tagen |
|---|---|---|
| **(IC)** | 500 | 0 |
| 0**Tefluthrin** | 0,8 | 40 |
| **(IC) + Tefluthrin (625:1)** | 500 + 0,8 | gef.*: 100<br>ber.**: 40 |

**Tabelle 13: (IC) + Spinosad**

| Wirkstoff | Wirtoffkonzentration [ppm] | Abtötungsgrad [%] nach 3 Tagen |
|---|---|---|
| **(IC)** | 500 | 0 |
| **Spinosad** | 0,16 | 60 |
| **(IC)+Spinosad (3125:1)** | 500 + 0,16 | gef.*: 95<br>ber.**: 60 |

**Tabelle 14: (IC) + Clothianidin**

| Wirkstoff | Wirkstoffkonzentration [ppm] | Abtötungsgrad [%] nach 3 Tagen |
|---|---|---|
| **(IC)** | 500 | 0 |
| **Clothianidin** | 4 | 85 |
| **(IC) + Clothianidin (125:1)** | 500 + 4 | gef.*: 100<br>ber.**: 85 |

**Tabelle 15: (IC) + Fipronil**

| Wirkstoff | Wirkstoffkonzentration [ppm] | Abtötungsgrad [%] nach 6 Tagen |
|---|---|---|
| **(IC)** | 500 | 20 |
| **Fipronil** | 0,8 | 0 |
| **(IC) + Fipronil (625:1)** | 500 + 0,8 | gef *: 55<br>ber.**: 20 |

**Tabelle 16: (IC) + Tebupirimfos**

| Wirkstoff | Wirkstoffkonzentration [ppm] | Abtötungsgrad [%] nach 6 Tagen |
|---|---|---|
| **(IC)** | 500 | 20 |
| **Tebupirimfos** | 0,8 | 0 |
| **(IC) + Tebupirimfos (625:1)** | 500 + 0,8 | gef.*: 45<br>ber.**: 20 |

**Tabelle 17: (IC) + (IIA)**

| Wirkstoff | Wirkstoffkonzentration [ppm] | Abtötungsgrad [%] nach 6 Tagen |
|---|---|---|
| **(IC)** | 500 | 20 |
| **(IIA)** | 100 | 30 |
| **(IC) + (IIA) (5:1)** | 500 + 100 | gef.*: 85<br>ber.**: 44 |

**Beispiel D**

**Myzus-Test**

Lösungsmittel: 7 Gewichtsteile Dimethylformamid

Emulgator: 2 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*), die stark von der Grünen Pfirsichblattlaus (*Myzus persicae*) befallen sind, werden durch Tauchen in die Wirkstoffzubereitung der gewünschten Konzentration behandelt. Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden. Die ermittelten Abtötungswerte verrechnet man nach der Colby-Formel (siehe Blatt 1).

Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen (gef.* = im Test gefundene Wirkung; ber.** = nach Colby berechnete Wirkung):

**Tabelle 18: (IC) + Tefluthrin**

| Wirkstoff | Wirktoffkonzentration [ppm] | Abtötungsgrad [%] nach 1 Tag |
|---|---|---|
| **(IC)** | 100 | 0 |
| **Tefluthrin** | 4 | 0 |

(fortgesetzt)

| Wirkstoff | Wirktoffkonzentration [ppm] | Abtötungsgrad [%] nach 1 Tag |
|---|---|---|
| **(IC) + Tefluthrin (25:1)** | 100+4 | gef.*: 30<br>ber.**: 0 |

**Tabelle 19: (IC) + Tebunirimfos**

| Wirktoff | Wirktoffkonzentration [ppm] | Abtötuntgsgrad [%] nach 6 Tagen |
|---|---|---|
| **Wirkstoff (IC)** | 100 | 0 |
| **Tebupirimfos** | 20 | 35 |
| **(IC) + Tebupirimfos (5:1)** | 100+20 | gef.*: 80<br>be.**: 35 |

**Tabelle 20: (IC) + (IIA)**

| Wirkstoff | Wirktoffkonzentration [ppm] | Abtötungsgrad [%] nach 6 Tagen |
|---|---|---|
| **(IC)** | 100 | 0 |
| **(IIA)** | 4 | 55 |
| **(IC) + (IIA) (25:1)** | 100+4 | gef.*: 65<br>ber.**: 55 |

**Patentansprüche**

**1.** Synergistische Mittel, **gekennzeichnet durch** den Gehalt an einer Wirkstoffkombination umfassend

(a) einen oder mehrere Wirkstoffe der Formel (I)

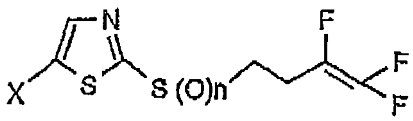 (I)

in welcher

*X* für Halogen, und
n für 0, 1 oder 2 steht,

und
(b) einen oder mehrere Wirkstoffe aus einer oder verschiedener der folgenden Gruppen (b1) bis (b7):

(b1) Aldicarb, Alanycarb, Aldoxycarb, Aminocarb, Bendiocarb, Benfuracarb, BPMC, Bufencarb, Butocarboxim, Carbaryl, Carbofuran, Carbosulfan, Cloethocarb, Ethiofencarb, Fenobucarb, Fenoxycarb, Flurathiocarb, Isoprocarb, Metam-sodium, Methiocarb, Methomyl, Metolcarb, Metolcarb, Oxamyl, Phosphocarb, Pirimicarb, Promecarb, Propoxur, Thiodicarb, Thiofanox, Trimethacarb, XMC, Xylylcarb;
(b2) Imidacloprid, Acetamiprid, AKD 1022, Clothianidin, Dinetofuran, Nitenpyram, Thiacloprid, Thiamethoxam;
(b3) Fipronil, Acetoprole, Ethiprole, Fenpyrxoximate, Vaniliprole;
(b4) Spinosad, Abamectin, Avermectin, Emamectin, Emamectin-Benzoate, Ivermectin, Milbemectin, Milbemycin, Moxidectin, Thuringiensin;

(b5) Tebupirimfos, Azamethiophos, Azinphos-ethyl, Azinphos-methyl, Bromophos-ethyl, Butathiofos, Cadusafos, Carbophenothion, Chlorethoxyfos, Chlorpyrifos, Chlorpyrifos, Chlorpyrifos-ethyl, Chlorpyrifos-methyl, Coumaphos, Cyanophos, Demeton, Demeton-S-methyl, Demeton-S-methyl-sulphon, Dialifos, Diazinon, Dichlofenthion, Dimethoate, Disulfoton, Ethion, Ethoprophos, Etrimfos, Fenitrothion, Fensulfothion, Fenthion, Flupyrazofos, Fonofos, Formothion, Fosmethilan, Iodofenphos, Iprobenfos, Isazofos, Isoxathion, Malathion, Meoarbam, Mesulfenfos, Methacrifos, Methidathion, Omethoate, Oxydemeton-methyl, Parathion-methyl, Phenthoate, Phorate, Phosalone, Phosmet, Phosphocarb, Phoxim, Pirimiphos-ethyl, Pirimiphos-, methyl, Profenofos, Prothiofos, Prothoate, Pyraclofos, Pyridaphenthion, Pyridathion, Quinalphos, Sulfotep, Sulprofos, Temephos, Terbufos, Thiatriphos, Thiometon, Triazophos, Vamidothion oder

(b6) Tefluthrin, Resmethrin, Acrinathrin, Allethrin (1R-Isomer), Alpha-Cypermethrin, Beta-, Cyfluthrin, Beta-Cypermethrin, Bifenthrin, Bioallethrin, Bioallethrin (S-cyclopentyl-Isomer), Bioethanomethrin, Biopermethrin, Bioresmethrin, Brofenprox, Chloethocarb, Chlovaporthrin, Cis-Cypermethrin, Cis-Resmethrin, Clocythrin, Cycloprothrin, Cyfluthrin, Cyhalothrin, Cypermethrin, Cyphenothrin (IR-trans-, somer), Deltamethrin, Dimefluthrin, Eflusilanate, Empenthrin (1R-Isomer), Esfenvalerate, Etofenprox, Fenfluthrin, Fenpropathrin, Fenpyrithrin, Fenvalerate, Flubrooythrinate, Flubrocythrinate, Flucythrinate, Flufenprox, Flufenprox, Flumethrin, Fluvalinate, Fubfenprox, Gamma-Cyhalothrin, Halfenprox, Imiprothrin, Kadethrin, Lambda-Cyhalothrin, Metofluthrin, MIT-800, Permethrin, Phenothrin (1R-trans-Isomer), Prallethrin, Profluthrin, Protrifenbute, Pyresmethrin, Pyrethrum, RU-12457, RU-15525, Silafluofen, Tau-Fluvalinate, Tetramethrin (1R-Isomer), Theta-Cypermethrin, Tralocythrin, Tralomethrin, Transfluthrin, Zeta-Cypermethrin;

(b7) Verbindung der Formel (IIA)

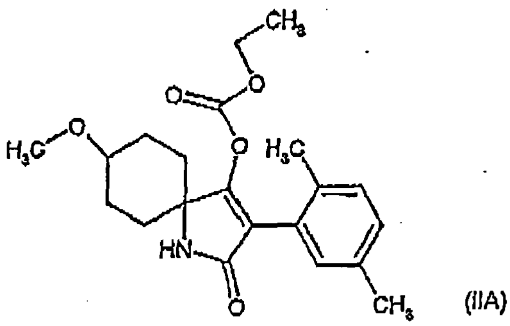

(IIA)

(Carbonsäure, 3-(2,5-dimethylphenyl)-8-methoxy-2-oxo-1-azaspiro[4.5]dec 3-en-4-yl-ethyl-ester, (9Cl));

2. Synergistische Mittel gemäß Anspruch 1, **dadurch gekennzeichnet, dass** sie eine oder mehrere Verbindungen der Formel (I) umfassen, in denen

X für Fluor, Chlor oder Brom steht, und
n für 0 oder 2 steht.

3. Synergistische Mittel gemäß Anspruch 1, **dadurch gekennzeichnet dass** sie eine oder mehrere Verbindungen der Formel (I) umfassen, in denen

n X für Fluor oder Chlor steht, und für 2 steht.

4. Sunergistische Mittel gemäß Anspruch 1, **dadurch gekennzeichnet; dass** sie als Wirkstoffe der Gruppe 1 eine oder mehrere der Verbindungen der Formel (IA), (IB) oder (IC)

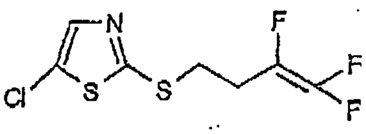 (IA) 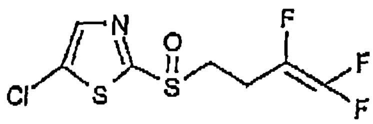 (IB)

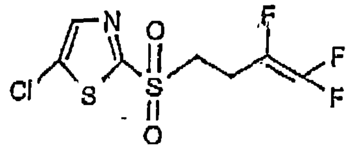

(IC)

umfassen,

**5.** Synergistische Mittel gemäß Anspruch 1, **dadurch gekennzeichnet, dass** sie als Wirkstoff der Gruppe 1 die Verbindungen der Formel (IC) umfassen.

**6.** Synergistische Mittel gemäß einem der Ansprüche 1 bis 5, **dadurch gekennzeichnet, dass** sie als Wirkstoffe der Gruppe 2 einen oder mehrere der folgenden Wirkstoffe umfassen;
Aldicarb, Clothianidin, Imidacloprid, Fipronil, Spinosad, Teffuthrin, Tebupirimfos, Verbindung der Formel (IIA)

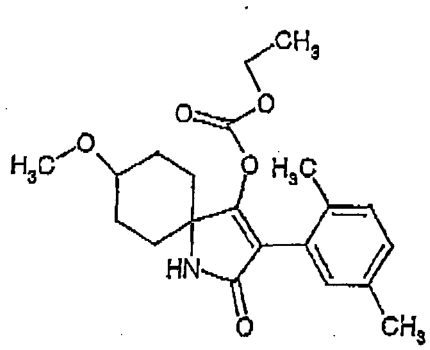

(IIA).

**7.** Verwendung von Mitteln gemäß einem der Ansprüche 1 bis 6 für die Herstellung von nematoziden, insektiziden oder akariziden Mitteln zur Bekämpfung von Schädlingen.

**8.** Verfahren für die Herstellung von nematoziden, insektiziden oder akariziden Mitteln zum Bekämpfen von Schädlingen, **dadurch gekennzeichnet, dass** man Mittel
gemäß einem der Ansprüche 1 bis 6 auf die Schädlinge und/oder ihren Lebensraum einwirken lässt.

**9.** Verfahren zum Herstellen von synergistischen Mitteln, **dadurch gekennzeichnet, dass** man ein Mittel gemäß einem der Ansprüche 1 bis 6 mit oberflächenaktiven Mitteln und/oder Streckmitteln vermischt.

## Claims

**1.** Synergistic means, **characterized by** the content of a combination of active agents, comprising

(a) one or more active agents with the Formula (I)

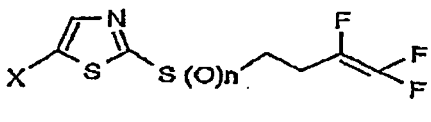

(I)

wherein

X represents halogen and
n represents 0, 1 or 2

and
(b) one or more active agents from one or various of the following groups (b1) through (b7):

(b1) aldicarb, alanycarb, aldoxycarb, aminocarb, bendiocarb, benfuracarb, BPMC, bufencarb, butocarboxim, carbaryl, carbofuran, carbosulphane, cloethocarb, ethiofencarb, fenobucarb, fenoxycarb, furathiocarb, isoprocarb, metam-sodium, methiocarb, methomyl, metolcarb, oxamyl, phosphocarb, pirimicarb, promecarb, propoxur, thiodicarb, thiofanox, trimethacarb, XMC, xylylcarb;
(b2) imidacloprid, acetamiprid, AKD 1022, clothianidin, dinetofuran, nitenpyram, thiacloprid, thiamethoxam;
(b3) fipronil, acetoprole, ethiprole, fenpyroximate, vaniliprole;
(b4) spinosad, abamectin, avermectin, emameotin, emamectin-benzoate, ivermectin, milbemectin, milbemycin, moxidectin, thuringiensin;
(b5) tebupirimfos, azamethiophos, azinphos-ethyl, azinphos-methyl, bromophos-ethyl, butathiofos, cadusafos, carbophenothion, chlorethoxyfos, chlorpyrifos, chlorpyrifos-ethyl, chlorpyrifos-methyl, coumaphos, cyanophos, demeton, demeton-S-methyl, demeton-S-methyl-sulphone, dialifos, diazinon, dichlofenthion, dimethoate, disulfoton, ethion, ethoprophos, etrimfos, fenitrothion, fensulfothion, fenthion, flupyrazofos, fonofos, formothion, fosmethilan, iodofenphos, iprobenfos, isazofos, isoxathion, malathion, meoarbam, mesulfenfos, methacrifos, methidathion, ornethoate, oxyderneton-methyl, parathion-methyl, phenthoate, phorate, phosalone, phosmet, phosphocarb, phoxim, pirimiphos-ethyl, pirimiphos-,methyl, profenofos, prothiofos, prothoate, pyraclofos, pyridaphenthion, pyridathion, quinalphos, sulfotep, sulprofos, temephos, terbufos, thiatriphos, thiometon, triazophos, vamidothion or
(b6) tefluthrin, resmethrin, acrinathrin, allethrin (1R-isomer), alpha-cypermethrin, beta-, cyfluthrin, beta-cypermethrin, bifenthrin, bioallethrin, bioallethrin (S-cyclopentyl-isomer), bioethanomethrin, biopermethrin, bioresmethrin, brofenprox, chloethocarb, chlovaporthrin, cis-cypermethrin, cis-resmethrin, clocythrin, cycloprothrin, cyfluthrin, cyhalothrin, cypermethrin, cyphenothrin (1R-trans-, somer), deltamethrin, dimefluthrin, eflusilanate, empenthrin (1R-isomer), esfenvalerate, etofenprox, fenfluthrin, fenpropathrin, fenpyrithrin, fenvalerate, flubrocythrinate, flucythrinate, flufenprox, flufenprox, flumethrin, fluvalinate, fubfenprox, gamma-cyhalothrin, halfenprox, imiprothrin, kadethrin, lambda-cyhalothrin, metofluthrin, MIT-800, permethrin, phenothrin (1R-trans-isomer), prallethrin, profluthrin, protrifenbute, pyresmethrin, pyrethrum, RU-12457, RU-15525, silafluofen, tau-fluvalinate, tetramethrin (1R isomer), theta-cypermethrin, tralocythrin, tralomethrin, transfluthrin, zeta-cypermethrin;
(b7) compound with the formula (IIA)

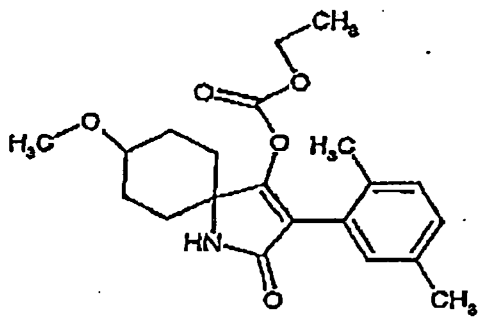

(IIA)

(carboxylic acid, 3-(2,5-dimethylphenyl)-8-methoxy-2-oxo-1-azaspiro[4.5]dec 3-en-4-yl-ethyl-ester, (9C1));

2. Synergistic means according to Claim 1, **characterized by** comprising one or more compounds of Formula (I), wherein

X represents fluorine, chlorine or bromine and
n represents 0 or 2.

3. Synergistic means according to Claim 1, **characterized by** comprising one or more compounds of Formula (I), wherein

X represents fluorine or chlorine and

n represents 2.

4. Synergistic means according to Claim 1, **characterized by** comprising, as active agents of group 1, one or more of the compounds of Formula (IA), (IB) or (IC).

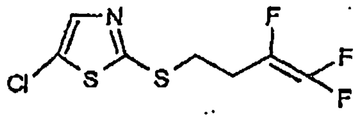 (IA) 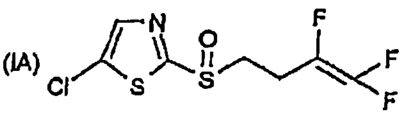 (IB)

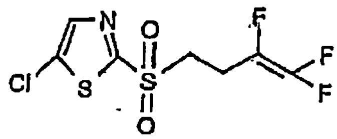 (IC)

5. Synergistic means according to Claim 1, **characterized by** comprising, as an active agent of group 1, the compounds of Formula (IC).

6. Synergistic means according to one of Claims 1 through 5, **characterized by** comprising, as active agents of group 2, one or more of the following active agents:

aldicarb, clothianidin, imidacloprid, fipronil, spinosad, tefluthrin, tebupirinfos, compound of Formula (IIA)

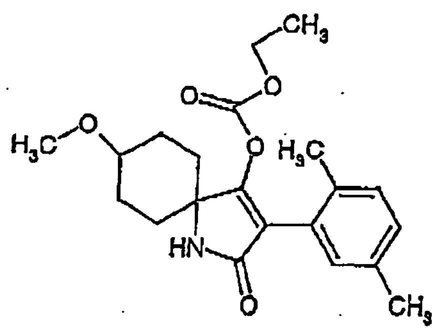 (IIA).

7. Use of means according to one of Claims 1 through 6 for the preparation of nematocidal, insecticidal or acaricidal means for fighting pests.

8. Method for the preparation of nematocidal, insecticidal or acaricidal means for fighting pests, **characterized in that** one lets means according to one of Claims 1 through 6 act on the pests and/or their habitat.

9. Method for the preparation of synergistic means, **characterized in that** one mixes a means according to one of Claims 1 through 6 with surfactants and/or extenders.

**Revendications**

1. Agents synergiques, **caractérisés par** la teneur en une combinaison de principes actifs comprenant

(a) un ou plusieurs principes actifs de formule (I)

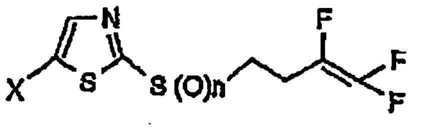

(I)

dans laquelle

X représente un halogène; et
n représente 0, 1 ou 2;

et

(b) un ou plusieurs principes actifs provenant d'un ou de plusieurs des groupes suivants (b1) à (b7) :

(b1) aldicarbe, alanicarbe, aldoxycarbe, aminocarbe, bendiocarbe, benfuracarbe, BPMC, bufencarbe, butocarboxime, carbaryle, carbofurane, carbosulfan, cloethocarbe, ethiofencarbe, fenobucarbe, fenoxycarbe, furathiocarbe, isoprocarbe, metam-sodium, methiocarbe, methomyle, metolcarbe, metolcarbe, oxamyle, phosphocarb, pirimicarbe, promecarbe, propoxur, thiodicarbe, thiofanox, trimethacarbe, XMC, xylylcarbe ;
(b2) imidaclopride, acetamipride, AKD 1022, clothianidine, dinetofurane, nitenpyram, thiaclopride, thiamethoxame ;
(b3) fipronile, acetoprole, ethiprole, fenpyroximate, vaniliprole ;
(b4) spinosade, abamectine, avermectine, emamectine, benzoate d'emamectine, ivermectine, milbemectine, milbemycine, moxidectine, thuringiensine ;
(b5) tebupirimfos, azamethiophos, azinphos-éthyle, azinphos-méthyle, bromophos-éthyle, butathiofos, cadusafos, carbophenothion, clorethoxyfos, chlorpyrifos, chlorpyrifos, chlorpyrifos-éthyle, chlorpyrifos-méthyle, coumaphos, cyanophos, demeton, demeton-S-méthyle, demeton-S-méthyle sulfone, dialifos, diazinone, dichlophenthion, dimethoate, disulfoton, ethion, ethoprophos, etrimfos, fenitrothion, fensulfothion, fenthion, flupyrazofos, fonofos, formothion, fosmethilan, iodofenphos, iprobenfos, isazofos, isoxathion, malathion, mecarbame, mesulfenfos, methacrifos, methidathion, omethoate, oxydemeton-méthyle, parathion-méthyl, phenthoate, phorate, phosalone, phosmet, phosphocarb, phoxim, pirimiphos-éthyle, pirimiphos-méthyle, profenofos, prothiofos, prothoate, pyraclofos, pyridaphenthion, pyridathion, quinalphos, sulfotep, sulprofos, temephos, terbufos, thiatrifos, thiometon, triazophos, vamidothion,
ou
(b6) tefluthrin, resmethrin, acrinathrin, allethrin (isomère 1R), alpha-cypermethrin, beta-, cyflothrin, beta-cypermethrin, bifenthrin, bioallethrin, bioallethrin (isomère S-cyclopentyle), bioethanomethrin, biopermethrin, bioresmethrin, brofenprox, chloethocarb, chlovaporthrin, cis-cypermethrin, cis-resmethrin, clocythrin, cycloprothrin, cyfluthrin, cyhalothrin, cypermethrin, cyphenothrin (1R-trans, somer), deltamethrin, dimefluthrin, eflusilanate, empenthrin (isomère 1R), esfenvalerate, etofenprox, fenfluthrin, fenpropathrin, fenpyrithrin, fenvalerate, flubrocythrinate, flubrocythrinate, flucythrinate, flufenprox, flufenprox, flumethrin, fluvalinate, fubfenprox, gamma-cyhalothrin, halfenprox, imiprothrin, kadethrin, lambda- cyhalothrin, metofluthrin, MIT-800, permethrin, phenothrin (isomère 1R-trans), prallethrin, profluthrin, protrifenbute, pyresmethrin, pyrethrum, RU-12457, RU-15525, silafluofen, tau-fluvalinate, tetramethrin (isomère 1R), theta-cypermethrin, tralocythrin, tralometrin, transfluothrin, zeta-cypermethrin ;
(b7) composé de la formule (IIA)

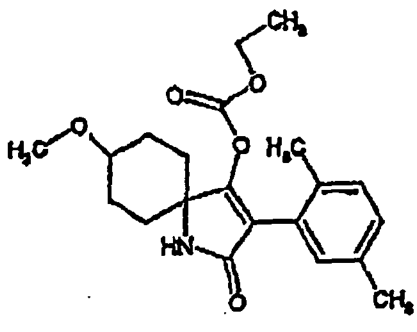

(IIA)

(acide carboxylique, 3-(2,5-diméthlphényl)-8-méthoxy-2-oxo-1-azaspiro[4,5]dec 3-èn-4-yl-éthyl-ester

(9Cl)).

2. Agents synergiques selon la revendication 1, **caractérisés en ce qu'**ils comprennent un ou plusieurs composés de la formule (I), dans lesquels

X représente un fluor, un chlore ou un brome, et
n représente 0 ou 2.

3. Agents synergiques selon la revendication 1, **caractérisés en ce qu'**ils comprennent un ou plusieurs composés de la formule (I), dans lesquels

X représente un fluor ou un chlore, et
n représente 2.

4. Agents synergiques selon la revendication. 1, **caractérisés en ce qu'**ils comprennent comme principes actifs du groupe 1, un ou plusieurs des composés des formules (IA), (IB) ou (IC)

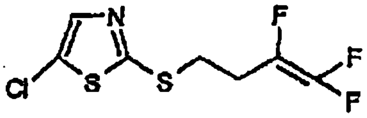
(IA)
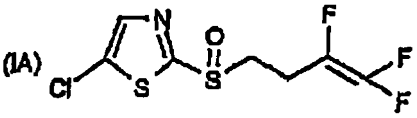
(IB)

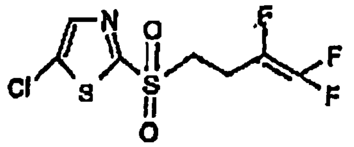
(IC)

5. Agents synergiques selon la revendication 1, **caractérisés en ce qu'**ils comprennent comme principe actif du groupe 1 les composés de la formule (IC).

6. Agents synergiques selon l'une quelconque des revendications 1 à 5, **caractérisés en ce qu'**ils comprennent comme principes actifs du groupe 2 un ou plusieurs des principes actifs suivants :

aldicarb, clothianidin, imidacloprid, fipronil, spinosad, tefluthrin, tebupirimfos, composé de la formule (IIA)

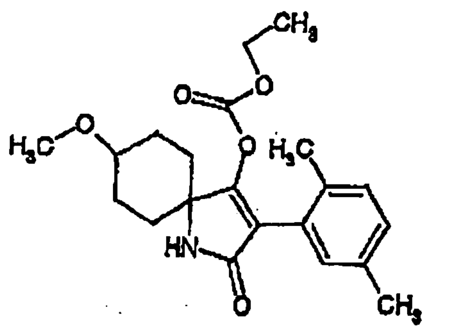
(IIA)

7. Utilisation d'agents selon l'une quelconque des revendications 1 à 6 pour la préparation de produits nématocides, insecticides ou acaricides pour la lutte contre les organismes nuisibles.

8. Procédé de préparation d'agents nématocides, insecticides ou acaricides pour la lutte contre les organismes nuisibles, **caractérisé en ce que** l'on fait agir des agents selon l'une quelconque des revendications 1 à 6 sur les organismes nuisibles et/ou sur leur espace vital.

**9.** Procédé de préparation d'agents synergiques, **caractérisé en ce que** l'on mélange un agent selon l'une quelconque des revendications 1 à 6 à des produits tensioactifs ou à des diluants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0102378 A1
- US 2758115 A
- US 3309266 A
- GB 1181657 A
- WO 9322297 A1
- WO 9310083 A1
- DE 2641343 A1
- EP 347488 A1
- EP 210487 A1
- US 3264177 A
- EP 234045 A2
- WO 9637494 A
- WO 9825923 A
- EP 0919542 A2


### In der Beschreibung aufgeführte Nicht-Patentliteratur


- **Ungerer.** *Chem. Ind.,* 1985, vol. 37, 730-732
- **Williams.** Antifouling Marine Coatings. Noyes, 1973
- **S.R. Colby.** *Weeds,* 1967, vol. 15, 20-22